# EUROPEAN PATENT APPLICATION

(11) **EP 4 445 857 A1**
(43) Date of publication of application: **16.10.2024**
(21) Application number: 23305550.8
(22) Date of filing: 12.04.2023
(51) Int. Cl.: A61B 34/30, A61B 34/00, B25J 13/02, G05G 9/047, G06F 3/033, A61B 17/56, A61B 90/00, G06F 3/0354

(54) **INPUT DEVICE FOR A COMPUTER-ASSISTED SURGICAL SYSTEM**

(71) Applicant: MinMaxMedical, 38400 Saint-Martin-d'Hères (FR)
(72) Inventor: DEHAN, Christophe, 38400 SAINT-MARTIN-D'HÈRES (FR); SEDNAOUI, Thomas, 38400 SAINT-MARTIN-D'HÈRES (FR); FOURNIER, Elie, 38400 SAINT-MARTIN-D'HÈRES (FR)
(74) Representative: Regimbeau

(57) **Abstract**

An input device comprises a static portion, a movable portion, and at least three posts extending between the static portion and the movable portion. Each post is fixed at a first end to the static portion and fixed at a second end, opposite the first end, to the movable portion. Each of the posts extends substantially parallel to each other and extends parallel to the axis of rotational freedom when the movable portion and the static portion are in a neutral position. The movable portion is displaceable along a first axis of translational freedom, a second axis of translational freedom, and an axis of rotational freedom. The movable portion and static portion are configured such that, when the movable portion is displaced relative to the static portion, any plane of the movable portion that is perpendicular to the posts in the neutral position and in which at least one of the second ends of the posts lies remains parallel to any plane of the static portion that is perpendicular to the posts in the neutral position and in which at least one of the first ends of the posts lies.

## Description

### TECHNICAL FIELD

The present disclosure relates generally to a handle including an input device adapted to guide movement of a component of a system with which the input device is operatively connected. More particularly, the present disclosure is related to an input device for guiding movement of a tool or robotic arm used in a computer-assisted surgical system.

### BACKGROUND

Computer-assisted surgical (CAS) systems may be guided by an operator. The operator is typically an orthopedic surgeon, a craniofacial surgeon, a dental surgeon, an ENT surgeon, or a neurosurgeon depending on the anatomical structure on which a procedure is performed. CAS systems may include a robot surgical platform that can assist the operator with positioning of surgical tools coupled to an end-effector of a robotic arm and performing surgical procedures within a patient body. CAS systems may be designed to restrict movement of the surgical tools to certain regions relative to a patient's anatomy such that the operation is minimally invasive and has increased accuracy.

In some CAS systems, the operator directly moves the surgical tool itself and the robotic arm reacts to the forces or motions of the surgical tool to constrain the surgical tool to some predefined rules or areas. In other CAS systems, the operator may directly act upon the robotic arm to guide movement of the surgical tool, and the robotic arm may transmit such detected movement to the surgical tool. In yet further CAS systems, the operator may direct movement of the robotic arm and/or surgical tool via a control device physically and/or operatively coupled to the robotic arm. Such control device may be a handle or joystick held by and manipulated by the operator. Examples of control devices for CAS systems are given in CN101999938B, EP4070752, and EP4070753.

CN101999938B describes a joystick having three degrees of freedom and configured to control a robotic arm. The joystick comprises three potentiometers configured to detect the displacement of two mutually perpendicular rotating axle located in a lower end of the handle to achieve a dual-axis operation and of a rotary knob located on the handle end. The joystick is associated with a control module configured to interpret the detected angulations of the joystick. As rotating shafts are used to move the joystick, such joystick is always moved along at least one rotational degree of freedom. The use of the joystick described in this document is counter-intuitive with respect to hand-eye coordination, as any given angulation given to such joystick by a user of the system is first interpreted by the control module to move the robotic arm according to either direction or angulation, depending on the mode selected through the mode selection switch.

EP4070752 and EP4070753 describe a handle for a computer-assisted surgery system having up to six degrees of freedom. The handle may include a fixed part and a movable part movable relative to the fixed part. One or more guides are provided between portions of the fixed part and the movable part to facilitate and limit the relative movement therebetween. Such guides may be, by way of non-limited example, a cylinder movable within a tube or ball bearings movable within a track. Other points of contacts may exist between the fixed part and the movable part of the handle. Over time, movement of the movable part relative to the fixed part results in wear on the guides and may shorten the usable lifetime of the handle. Accordingly, there is a need to minimize wear in handles.

### BRIEF SUMMARY

An objective of the present disclosure is to provide an input device for directing movement of a robotic arm and/or surgical tool solving one or more of the above-mentioned drawbacks.

The input device comprises a static portion, a movable portion, and at least three posts extending between the static portion and the movable portion. The movable portion is displaceable relative to the static portion along at least two degrees of freedom and up to three degrees of freedom. Each post is fixed at a first end to the static portion and fixed at a second end, opposite the first end, to the movable portion. Each of the posts extends substantially parallel to each other and extends parallel to an axis of rotational freedom when the movable portion and the static portion are in a neutral position. The movable portion is displaceable along a first axis of translational freedom, a second axis of translational freedom, and the axis of rotational freedom. The movable portion and static portion are configured such that, when the movable portion is displaced relative to the static portion, any plane of the movable portion that is perpendicular to the posts in the neutral position and in which at least one of the second ends of the posts lies remains parallel to any plane of the static portion that is perpendicular to the posts in the neutral position and in which at least one of the first ends of the posts lies.

Certain preferred but non-limiting features of the input device described above are the following, taken individually or in combination:
each post is elastically deformable;
the device further comprises a stiffener provided about the first end of each post, the stiffener having an abutment surface contactable by the posts when the movable portion is displaced from the neutral position;
the stiffener is adjustably coupled to one of the static portion and the movable portion;
the abutment surface comprises an arcuate surface and/or a stepped surface;
the device further comprises a pin coupled at a first end to one of the static portion and the movable portion and an aperture formed in the other of the static portion and the movable portion, a second end of the pin is received in the aperture;
the device further comprises a damping element coupled to one of the pin proximate the second end or on a surface of the static portion or the movable portion defining the opening;
when viewed in a plane perpendicular to the posts in the neutral position, the opening has a regular or irregular shape defined by curved surfaces of the static portion;
the device comprises four posts;
a width or diameter of each post measured along a first axis parallel to the first translational axis is different from a width or diameter measured along a second axis parallel to the second translational;
a width or diameter of each post measured along a first axis parallel to the first translational axis is equal to a width or diameter measured along a second axis parallel to the second translational axis;
each of the posts is equal in length;
the device further comprises a system for tracking displacement of the movable portion relative to the static portion, which system comprises a plurality of sensors coupled to one of the static portion and the movable portion and at least one magnetic source coupled to the other of the static portion and the movable portion, wherein the plurality of sensors are configured to measure an intensity of a magnetic field emitted by the at least one magnetic source;
the plurality of sensors are unidirectional sensors configured to measure an intensity of the magnetic field of the magnetic source in a direction parallel to the rotational axis; and/or
the movable portion is displaceable relative to the static portion along two degrees of translational freedom and one degree of rotational freedom.

Another objective of the present disclosure is providing a computer-assisted surgical system comprising a robotic arm with a plurality of motorized joints and a handle for controlling the robotic arm in which the input device described above is located. The handle comprises a static part coupled to the robotic arm and a movable part coupled to the static part and configured to be displaced relative to the static part. The static portion of the input device is disposed within the static part of the handle and the movable portion is disposed within the movable part of the handle.

### BRIEF DESCRIPTION OF THE DRAWINGS

The illustrations presented herein are not meant to be actual views of any particular component, device, or system, but are merely idealized representations which are employed to describe embodiments of the present invention. Other features, goals, and advantages of the present invention will appear more clearly upon reading of the detailed description that follows and with references to drawings provided by way of non-limiting examples wherein:
FIG. 1 illustrates, schematically, a computer-assisted surgery system;
FIGS. 2 and 3 are perspective and side views, respectively, of a handle;
FIG. 4A is a cross-sectional view of the handle and FIG 4B is an enlarged, partial view of the handle as shown in FIG. 4A;
FIGS. 5 and 6 are side and perspective views, respectively, of an input device within the handle of FIGS. 2-4B;
FIGS. 7 and 8 are schematic illustrations of the input device in a neutral position and displaced position, respectively;
FIGS. 9A and 9B are schematic cross-sectional views of posts of the input device;
FIGS. 10 and 11 are plots of the force required to be exerted by a user on the handle to translate a movable part of the input device a given distance relative to a static part of the input device; and
FIGS. 12 and 13 schematically illustrate a displacement sensor system of the input device.

### DETAILED DESCRIPTION

As used herein, relational terms, such as "first," "second," "top," "bottom," "upper," "lower," "over," "under," "on," "interior," "exterior," and the like, are used for clarity and convenience in understanding the disclosure and accompanying drawings and do not connote or depend on any specific preference, orientation, or order, except where the context clearly indicates otherwise.

As used herein, the term "and/or" includes any and all combinations of one or more of the associated listed items.

As used herein, the term "configured" refers to a size, shape, material composition, material distribution, orientation, and arrangement of one or more of at least one structure and at least one apparatus facilitating operation of one or more of the structure and the apparatus in a pre-determined way.

As used herein, the terms "part" or "portion" as used in "static part," "movable part," "static portion," and "movable portion" herein are both singular and plural. For instance, a static part of a handle or a static portion of an input device described herein may be formed by a single component or by multiple components or pieces assembled together in a manner that the component(s) or pieces are fixed relative to the structure to which the handle or input device is coupled. Similarly, a movable part of a handle or a movable portion of an input device described herein may be formed by a single component or by multiple components or pieces assembled together in a manner that the component(s) or pieces are fixed relative to each other but are movable relative to a corresponding static part or portion.

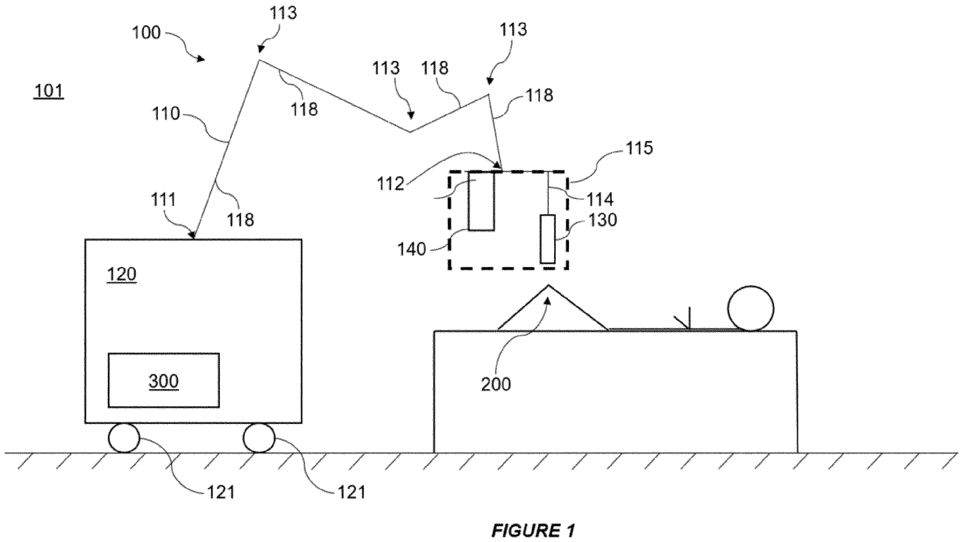

FIG. 1 schematically illustrates a computer-assisted surgery system 100 being represented in an operating room 101. As used herein, the words "computer-assisted surgery system 100" and "system 100" are used interchangeably and without any distinction. The computer-assisted surgery system 100 comprises at least one robotic arm 110 extending between a first end 111 and a second end 112. The first end 111 may be attached to a base 120 of the computer-assisted system 100. The second end 112 forms a flange of the robotic arm 110 to which an end-effector 115 is fixed. As described in further below, the end-effector 115 may also comprise part of the robotic arm 110 located before its flange, that is to say between the base 120 and the flange.

The base 120 may be a movable base and comprises a mechanism, such as wheels 121, permitting movement of the base 120 within the operating room 101. Accordingly, the user of the system 100 is able to easily move the base 120 depending on the treatment to be performed. In other non-illustrated embodiments, the base may be movable along rails or it may alternately be a fixed base, or it may be arranged on any suitable device. The base 120 further comprising mechanisms configured to lock the position of such base during the planned treatment.

At least three motorized joints 113 are formed between the first end 111 and the second end 112 of the robotic arm 110. More than three motorized joints 113 may be formed between the first end 111 and the second end 112 of the robotic arm 110. As used herein, the term "motorized joint" refers to a joint driven by its own motor, such joint being configured to be subjected to a linear deformation or to an angular deformation, within the scope of the invention. In other words, if *n* joints are formed between the first end 111 and the second end 112 of the robotic arm 110, the robotic arm 110 comprises n motors, each of which being configured to drive one of the joints.

The robotic arm 110 comprises several segments 118. As illustrated in FIG. 1, each segment 118 is separated from the next one by at least one motorized joint 113. As used herein, the term "end-effector" designated as element 115 refers to the last segment 118 of the robotic arm 110, that is to say the segment of such robotic arm 110 positioned the farthest from the base 120 of the system 110, or the second to last segment 118 of the robotic arm 110. In some non-illustrated embodiments, the end-effector 115 may comprise one or several motorized joint(s) and the end-effector 115 may thus comprise at least two segments 118 of the robotic arm 110. The end-effector 115 may itself encompass one or several additional segments and related degrees of freedom, such as a power tool in translation, irrigation and suction apparatus, safety observation instrumentation, etc.

The robotic arm 110 illustrated in FIG. 1 is a serial robotic arm, but this robotic arm could be a parallel robotic arm, or a combination thereof within the scope of the invention. In some embodiments, the robotic arm may present at least six motorized joints.

The computer-assisted surgery system 100 also comprises at least one surgical tool 130. The surgical tool 130 is configured to be used to perform a treatment on an anatomical structure 200.

The system 100 also comprises a handle 140. The surgical tool 130 and the handle 140 may both be attached to the robotic arm 110. The handle 140 may also be attached to any segment 113 of the robotic arm 110. In an exemplary embodiment as illustrated in FIG. 1, the handle 140 and the surgical tool 130 may be considered to form part of the end-effector 115 of the robotic arm 110. As the surgical tool 130 and the handle 140 form part of the end-effector 115, it is understood, from what has been described above referring to the end-effector, that such surgical tool 130 and such handle 140 may be arranged on the last segment 118 of the robotic arm 110 or on the second to last segment 118 of such robotic arm 110. According to the illustrated embodiment, the surgical tool 130 and the handle 140 are fixed to the flange of the robotic arm 110. For instance, the surgical tool 130 may be fixed to such flange by a shaft 114. Alternately, the surgical tool 130 may be directly mounted on the flange of the robotic arm 110. According to a non-illustrated embodiment, the handle 140 may be directly fixed to the surgical tool 130.

By way of example and not limitation, the surgical tool 130 may comprise at least one power tool configured to drive a tool. The tool may for instance be a cutting tool, such as a saw, a drill, a reamer or a burr. Alternatively, the surgical tool 130 may be a cutting guide or an insertion guide. Other known surgical tool 130 may be used without departing from the scope of the present invention depending upon the procedure to be performed on a given anatomical structure. Any surgical tool that is configured to act on or to treat an anatomical structure may be attached to the robotic arm within the scope of the invention. By way of further non-limiting examples, the surgical tool may be also an ultrasonic bone scalpel, a bone shaver, a laser that may cuts tissues or bones, a knife, a lancet, a cryosurgery probe, any radiofrequency tool, a microwave probe, a waterjet device, an ultrasonic probe, or a screwdriver.

By way of example and not limitation, the anatomical structure 200 may be a bone of a patient. According to the illustrated embodiment, the anatomical structure 200 is for example a tibial bone of the patient. As previously mentioned, the illustrated embodiment is only one example of how to carry the invention and the anatomical structure could be any other anatomical structure of said patient within the scope of the invention. For instance, this anatomical structure could be a femur bone, a shoulder scapula or humerus, a maxillo-facial bone, a small hand or foot bone such as metatarsal bone or talus, a vertebra, a pelvis, a tooth or mandible, a skull, a brain, and so forth.

The system 100 is particularly well suited to be used during orthopedic, ENT, cranio-facial, dental surgeries, or neurosurgery in which the degrees of motion to be imparted by the user is analogous to the degrees of freedom of the input device 10 such that the natural human coordination is respected. In this way, use of the system 100 is more intuitive to the user. By way of non-limiting, example, the tool 130 may be an oscillating bone saw to be manipulated within a cut plane having two translations with an orientation of the blade of the bone saw is managed by rotation of the input device 10.

The system 100 further comprises at least one control unit 300 configured to compute and instruct movement(s) to the motorized joints 113 of the robotic arm 110 which holds the surgical tool 130. The instructions configured to be sent by the control unit 300 are computed by the control unit 300 based on several inputs including movements imparted by a user onto the handle 140 described below. The control unit 300 can, for instance, comprise one or more microprocessor, one or more random access memory (RAM) and/or one or more read-only memory (ROM), one or more calculators, one or more computers and/or one or more computer programs. The computer program(s) comprise code instructions to compute the needed instructions to be sent to the motorized joints 113 of the robotic arm. 110. In addition, the control unit 300 may include other devices and circuitry for performing the functions described herein such as, for example, a hard drive, input/output circuitry, and the like. The input/output circuitry can be adapted to treat digital and/or analog signals.

According to the illustrated embodiment, the control unit 300 is integrated within the base 120 but such control unit 300 could be remote from the base 120 or elsewhere within the system 100 without departing from the scope of the invention. The surgical tool 130 comprises a tool center point which forms the point of such surgical tool around which are applied the rotations requested by the user - and modified by other inputs. This tool center point may be modified during the course of the treatment.

The handle 140 may comprise an activation mechanism 150. The activation mechanism 150 is provided to prevent unintended displacement of the handle 140. According to the embodiments illustrated, the activation mechanism 150 is formed by at least one finger activated button, acting as a trigger or as a variable command.

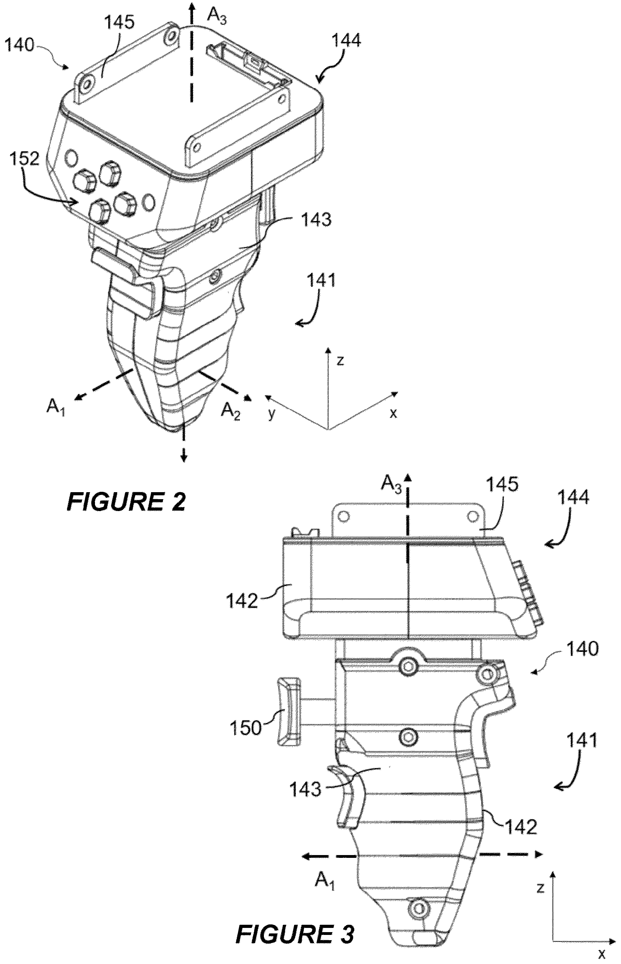

With reference to FIGS. 2 and 3, the handle 140 comprises a static part 144 that may be attached to the robotic arm 110 such that the static part 144 is not movable (e.g., is fixed, is static) relative to the segment 113 of the robotic arm 110 to which the handle 140 is attached. The handle 140 may be coupled to the robotic arm 110 by a bracket 145 or other attachment means. The handle 140 also comprises a movable part 141 that is movable (e.g., displaceable) relative to the static part 144 and relative to the robotic arm 110.

The movable part 141 exhibits at least two degrees of freedom and, preferably, three degrees of freedom. The degrees of freedom include (e.g., comprise, consist of) two degrees of translational freedom and one degree of rotational freedom. The coordinate system for the three degrees of freedom of the movable part 141 is illustrated in FIG. 2. The handle 140 is translatable forward-backward along the x-axis, translatable side-to-side along the y-axis, and rotatable (e.g., yaw) about the z-axis (e.g., normal axis).

The static part 144 and the movable part 141 are defined by different portions of a housing 142 of the handle 140. At least some portion of the housing 142 of the movable part 141 is configured to be gripped by a user's hand such portion of the handle 140 being referred to as a gripping part 143.

The handle 140 may contain several switches 152 to control various modes of the system that can be activated with a finger or a thumb depending on the position of the concerned switch with respect to the handle 140.

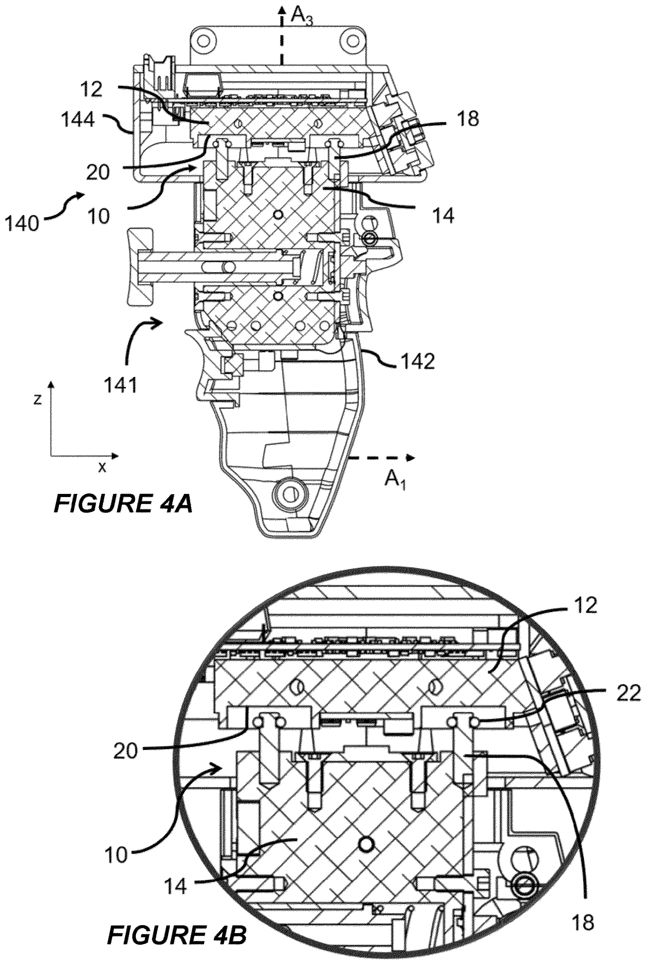

The handle 140 further comprises an input device 10. The input device 10 is shown within the handle 140 in the cross-sectional views of FIGS. 4A and 4B.

The input device 10 is a device from which relative positions and relative displacements of the static part 144 and the movable part 141 are measured. The input device 10 is also a device with which relative positions and relative displacements of the static part 144 and the movable part 141 may be controlled (e.g., limited) such that the movable part 141 has a predefined range of displacement relative to the static part 144.

As best illustrated in the enlarged view of FIG. 4B, a gap is provided between the static part 144 and the movable part 141. This arrangement of the static part 144 and the movable part 141 prevents wear at an interface therebetween so as to extend the functional life of the input device 10 and the handle 140 in which the device 10 is located and prevents friction generation at the interface which may lead to a stick-slip effect. Further, it is not necessary to provide guiding means such as those described in EP4070752 and EP4070753 at the interface between the static part and the movable part.

The input device 10 comprises a static portion 12 and a movable portion 14. The static portion 12 is static relative to the segment 113 of the robotic arm 110 to which it is attached, and the movable portion 14 is movable relative to the static portion 12 and the robotic arm 110.

The static portion 12 is located within the static part 144 of the handle 140. The static portion 12 is coupled to one or more structures within the static part 144 such that the static portion 12 of the input device 10 and the static part 144 of the handle 140 are not displaced relative to each other.

The movable portion 14 is located within the movable part 141 of the handle 140. As seen in FIGS. 4A and 4B, the movable portion 14 may also be partially located within the static part 144 of the handle 140. The movable portion 14 is coupled to one or more structures within the movable part 141 of the handle 140 such that the movable portion 14 of the input device 10 is static relative to the movable part 141 of the handle 140. Accordingly, in operation, when the user grasps the handle 140 and displaces the movable part 141, the movable portion 14 is displaced in a corresponding manner with displacement of the movable part 141.

While each of the movable portion 14 and the static portion 12 have been schematically illustrated in the figures as a single piece, the movable portion 14 and/or static portion 12 may be formed of a single piece or may be formed of two or more pieces.

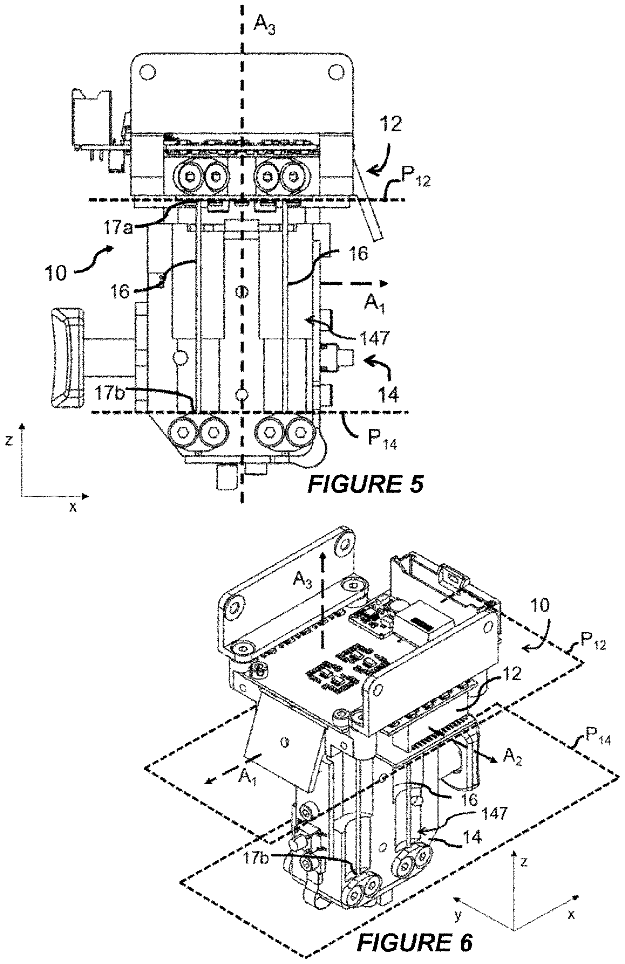

As illustrated in FIGS. 5-8, the input device 10 comprises a plurality of posts 16 that extend between the movable portion 14 and the static portion 12. Each post 16 extends between a first end 17a and a second, opposite end 17b. The first end 17a is coupled to the static portion 12, and the second end 17b is coupled to the movable portion 14.

A first end 17a of at least one post 16 lies in a plane P₁₂ of the static portion 12 which plane P₁₂ is perpendicular to the posts 16 in the neutral position. In some embodiments, the first end 17a of more than one post 16 lies in the same plane P₁₂. A second end 17b of at least one post 16 lies in a plane P₁₄ of the movable portion 14 which plane P₁₄ is perpendicular to the posts 16 in the neutral position. In some embodiments, the second end 17b of more than one post 16 lies in the same plane P₁₄.

Each post 16 is equal in length (i.e., a dimension measured from the first end 17a to second end 17b). In some embodiments, the first ends 17a of each post 16 lie in a common plane P₁₂, and the second ends 17b of each post 16 lies in a common plane P₁₄. In other embodiments, the first ends 17a of the posts 16 may each lie in different, but parallel, planes P₁₂ of the static portion 12 and the second ends 17b of the posts 16 may each lie in different, but parallel, planes P₁₄ of the movable portion 14. In the neutral position, the planes P₁₂, P₁₄ are parallel to each other.

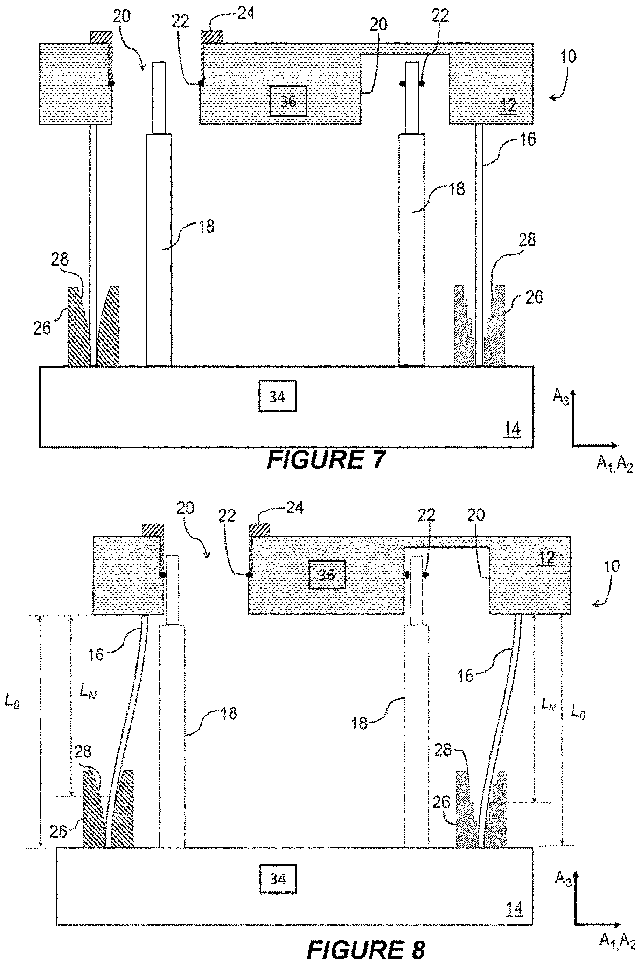

The posts 16 are configured to deform when the movable portion 14 is displaced relative to the static portion 12. In particular, the posts 16 are configured to elastically deform. Such deformation is schematically illustrated by comparing FIGS. 7 and 8.

In operation, the input device 10 is biased to the neutral position. In some embodiments, the posts 16 may be made of a material that urges the movable portion 14 to the neutral position. Accordingly, the posts 16 are configured to urge the movable portion 14 to the neutral position.

Within the handle 140, the movable portion 14 may be a body to which one or more of the components of the handle 140 as previously described herein are attached. In some embodiments, the movable portion 14 may comprise one or more recesses 147 having a surface to which the posts 16 are coupled at the second end 17b thereof.

The movable portion 14 and the static portion 12 are separated by a gap. As explained with reference to the movable part 141 and the static part 144, this arrangement of the movable portion 14 and the static portion 12 prevents wear, friction generation, and stick-slip effect at an interface therebetween, which extends the life of the input device 10 and the handle 140 in which it is located.

Like the movable part 141, the movable portion 14 of the input device 10 exhibits at least two and, preferably, three degrees of freedom. The three degrees of freedom include freedom of translational movement along two axes and freedom of rotational movement along one axis. The coordinate system for the three degrees of the input device 10 is the same as the coordinate system for movement of the handle 140. Accordingly, the movable portion 14 is translatable forward-backward along a first axis of translation A₁ in the x-direction, translatable side-to-side along a second axis of translation A₂ in the y-direction, and rotatable (e.g., yaw) about an axis of rotation A₃ extending in the z-direction. The axes of translation A₁, A₂ lie in the plane P₁₄. The axis of rotation A₃ is normal to the plane P₁₄.

The input device 10 is configured such that, when the movable portion 14 is displaced relative to the static portion 12, the plane P₁₄ of the movable portion 14 remains parallel to the plane P₁₂ of the static portion 12.

The input device 10 may be further configured such that the movable portion 14 is not translatable along the z-axis. The input device 10 further configured such that the movable portion 14 is not rotatable about the first translational axis A₁ nor about the second translational axis A₂. Accordingly, the input device 10 exhibits up to three degrees of freedom.

FIG. 7 illustrates the input device 10 in a neutral position, and FIG. 8 illustrates the input device 10 when the movable portion 14 has been displaced relative to the static portion 12 as indicated by the arrow. In the neutral position, the posts 16 are undeformed such that each of the posts 16 extends substantially parallel to each other between the static portion 12 and the movable portion 14.

With continued reference to FIGS. 7 and 8, the input device 10 may further comprise a plurality of stiffeners 26. Each stiffener 26 is associated with a respective post 16. The stiffener 26 has an abutment surface 28 facing the post 16 about which the stiffener 26 is arranged. The stiffener 26 is provided such that the posts 16 may contact the abutment surface 28 when the movable portion 14 is displaced relative to the static portion 12. Each stiffener 26 is arranged to at least partially (e.g., partially, entirely) enclose a portion of each post 16 proximate to one of the ends 17a, 17b. As illustrated in FIGS. 7 and 8, the stiffeners 26 are located adjacent the second end 17b coupled to the movable portion 14.

The posts 16 may be separate from (e.g., not in contact with) the stiffener 26 when the input device 10 in the neutral position. In the neutral position, the posts 16 extend substantially parallel to the rotational axis A₃ and substantially perpendicular (e.g., normal) to a translational plane in which the axes A₁ and A₂ of translational movement lie. Some deviation of the posts 16 from parallel is acceptable without negatively impacting the function of the input device 10.

Each post 16 has an initial length L₀ measured in a direction parallel to the rotational axis A₃ and between the first end 17a and the second end 17b. When the movable portion 14 is displaced, a portion of the post 16 may contact a portion of the abutment surface 28 of the stiffener 26. When the post 16 contacts the abutment surface 28 of the stiffener 26, the effective length L*ₙ* of the post 16 is modified. The effective length L*ₙ* refers to the length of a portion of the post 16 measured between the point at which the post 16 is coupled to the static portion 12 and a point of contact 25 of the post 16 with the abutment surface 28 of the stiffener 26. The effective length L*ₙ* is measured in a direction parallel to the rotational axis A₃.

The abutment surface 28 is configured such that the post 16 becomes increasingly engaged with the abutment surface 28 as the movable portion 14 is displaced further from the neutral position. The effective length L*ₙ* of the post 16 decreases as the post 16 increasingly contacts the abutment surface 28. Modifying the effective length L*ₙ* of the post 16 results in a modification to the translational stiffness of the post 16. In particular, decreasing the effective length of the post 16 increases the translational stiffness of the post 16.

In operation, when a user grips the handle 140 and displaces the handle 140 by applying a force thereto, the movable part 141 and the movable portion 14 are displaced. As the movable portion 14 is displaced, the post 16 is deformed and a portion of the post 16 abuts against the abutment surface 28 of the stiffener 26, thus increasing the translational stiffness and torsional stiffness of the post 16. When the translational stiffness and the torsional stiffness of the post 16 increases, the user must apply an increasing amount of force onto the handle 140 to continue to displace the movable part 141 along the translational axes A₁, A₂ and/or about the rotational axis A₃.

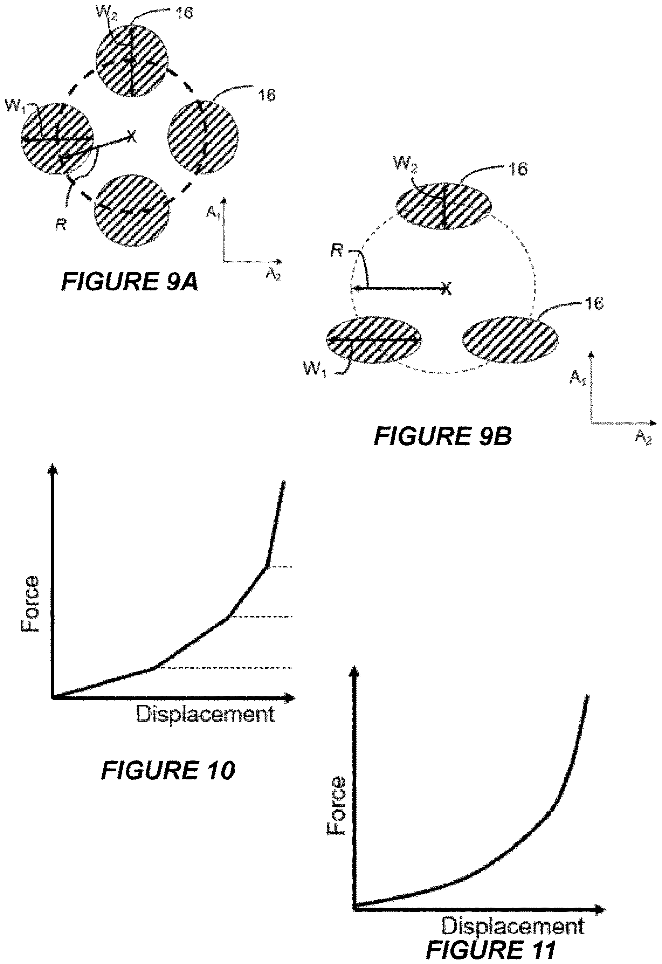

FIGS. 7 and 8 illustrate two potential embodiments of the stiffener 26. FIGS. 10 and 11 are graphs demonstrating the relationship between the amount of displacement of the movable portion 14 relative to the static portion 12 such that the posts 16 engage with the stiffener 26 and the force that must be applied to the movable part 141 including the movable portion 14 to continue displacement thereof.

The rate at which the translational stiffness and torsional stiffness of the post 16 increases depends upon the shape of the abutment surface 28 as will be understood from the following exemplary embodiments. The rate at which the amount of applied force or torque must be increased by the user to continue displacement may be varied based upon the configuration of the abutment surface 28. This increasing force or torque haptically conveys to the user gripping the handle 140 that motion in the direction of the applied force may be reaching the end of its possible range of movement.

According to a first exemplary embodiment of the stiffener 26 illustrated in FIGS. 7 and 8, the abutment surface 28 is stepped. FIG. 10 illustrates the change in force with increasing displacement along one of the translational axes A₁, A₂ for a stepped surface. For the stepped abutment surface 28, the translational stiffness of the posts 16 is modified in a manner that the force required for continued displacement increases in a piecewise linear manner as the amount of displacement along a translational axis A₁, A₂ increases. When the post 16 contacts a corner of each step, the rate at which the translational stiffness increases changes.

According to a second exemplary embodiment of the stiffener 26 illustrated in FIGS. 7 and 8, the abutment surface 28 is an arcuate (e.g., curved) surface. In three-dimensions, the space defined by the interior of the stiffener 26 may be a curved cone. FIG. 11 illustrates the change in force with increasing displacement along one of the translational axes A₁, A₂ for the curved surface. For a curved abutment surface 28, the stiffness of the posts 16 is modified in a non-linear manner.

The skilled person would recognize that other configurations of the abutment surface 28 are possible and that such configurations may be selected based the desired rate at which force and/or torque must be increased by the user to continue displacement and based on the desired range of motion of the handle 140. By way of further example, the abutment surface 28 could include both a stepped and curved surface

The input device 10 may be configured such that the stiffener 26 is adjustably coupled to one of the movable portion 14 and the static portion 12. For example, the stiffener 26 may be located within a recess formed in one of the movable portion 14 or static portion 12 to which the stiffener 26 is coupled. The stiffener 26 and recess may be configured such that the stiffener 26 is translatable along an axis parallel to the rotational axis A₃. Adjusting the stiffener 26 changes the relative position of the abutment surface 28 and the posts 16 and, accordingly, adjusts the point at the posts 16 would contact the abutment surface 28.

One or more design parameters and/or properties of the individuals posts 16 or the assembly of the plurality of posts 16 may be selected to achieve a pre-determined translational and/or torsional stiffness to the posts 16.

With reference to the individual posts 16, the translational stiffness of the posts 16 may be a function of one or more of cross-sectional shape, cross-sectional dimensions, and/or material composition of the posts 16.

The cross-sectional shape of the posts 16 refers to the shape of the posts 16 taken in a plane that is perpendicular to the rotational axis A₃ and parallel to the translational axes A₁, A₂. The cross-sectional shape may be one of a circle, an oval, a square, or a rectangle. In some embodiments, each of the posts 16 of the input device 10 has the same cross-sectional shape.

The cross-sectional dimensions of the posts are those dimensions measured in a plane that is perpendicular to the rotational axis A₃ and parallel to the translational axes A₁, A₂. Cross-sectional shape and dimensions of the posts 16 may be selected as a function of the pre-determined torsional and translational stiffness of the posts.

The width or diameter of the cross-section of the post 16 may be selected to impart a given translational stiffness and torsional stiffness to the post 16. Assuming all other parameters are unchanged, the translational stiffness and the torsional stiffness of a given post 16 increases with increasing width or diameter. In some embodiments, the post 16 may have the same width or diameter along a first axis parallel to the first translational axis A₁ and a second axis parallel to the second translational axis A₂, as illustrated in the exemplary cross-sectional view of FIG. 9A. In other embodiments, the post 16 may have a width or diameter Wi measured along the first axis parallel to the first translational axis A₁ that is different from a width or diameter W₂ measured along the second axis parallel to the second translational axis A₂, as illustrated in the exemplary cross-sectional view of FIG. 9B. The translational stiffness will be greater along the axis having the greater width or diameter. The cross-sectional shape and/or dimensions may, therefore, be selected such that the user may more easily displace the handle 140 along one of the first translational axis A₁ and the second translational axis A₂.

Additionally, assuming all other parameters are unchanged, modifying the cross-sectional dimensions of the post 16 results in a variation of the translational and torsional stiffness of the post 16. In particular, increasing the width or diameter of the post 16, increases both the translational stiffness and the torsional stiffness of the post 16. Conversely, decreasing the width or diameter of the post 16, decreases both the translational stiffness and the torsional stiffness of the post 16.

The posts 16 are formed of a material that is rigid along the z-axis (extending longitudinally between the respective ends 17a, b thereof). The posts 16 may be formed of a metallic material including, but not limited to, spring steel, stainless steel, nitinol, titanium, a shape memory metallic alloy, or superelastic alloy. The posts 16 may also be formed of a composite material such as a fiber reinforce plastic.

With reference to the plurality of posts 16 of the input device 10, the translational stiffness and the torsional stiffness may be a function of the number of posts 16 and the arrangement of the posts 16 relative to a centroid of the plurality of posts 16.

The number of posts 16 of the input device 10 may be selected to impart a defined torsional stiffness to the input device 10. At a minimum, the input device 10 includes three posts 16. As shown in FIG. 9A, the input device 10 comprises four posts 16, and, as shown in FIG. 9B, the input device 10 comprises three posts 16. Increasing the number of posts 16 increases the torsional stiffness of the input device 10 and decreasing the number of posts 16 decreases the torsional stiffness of the input device 10, assuming all other parameters are unchanged. The number of posts 16 of the input device 10 affects proportionally the translational stiffness of the input device 10.

The arrangement of the posts 16 relative to a centroid (indicated by the X in FIGS. 9A and 9B) may be selected to impart a pre-determined torsional stiffness to the input device 10. Each post 16 is located at a distance R from the centroid. Assuming all other parameters are unchanged, increasing the distance R increases the torsional stiffness of the input device 10, and decreasing the distance R decreases the torsional stiffness of the input device 10. Translational stiffness is independent of the distance R.

In view of the foregoing, the design of the individual posts 16 and the collective arrangement of the posts 16 may be selected such that the input device 10 has a pre-determined translational stiffness, torsional stiffness, and/or ratio of translational stiffness to torsional stiffness. The translational stiffness and torsional stiffness may be selected based on the desired displacement of the robotic arm 110 or surgical tool 130 with which the input device 10 is associated. By way of non-limiting example, the individual posts 16 and/or the collective arrangement of the posts 16 may be selected such that the translational stiffness is less than the torsional stiffness so as to favor translational movement of the handle 140. Advantageously, the lateral translation can be stiffer than the axial translation. In this way, the user is able to more easily guide displacement of the surgical tool along a preferred path.

While the posts 16 have been described as individual elements, the posts 16 may be integrally formed. In such an embodiment, the input device 10 may comprise a tubular structure. The tubular structure may comprise an annular first end coupled to the movable portion 14 and an annular second end coupled to the static portion 12. The annular first and second ends are coupled together by a discontinuous cylindrical wall. The cylindrical wall is discontinuous by virtue of a plurality of cutaways. The discrete portions of the cylindrical wall serve as posts 16 as described herein. In such an embodiment, the cross-sectional dimensions of the posts 16 is equal to a thickness of the cylindrical wall, the effective length of the posts 16 is the distance of the discrete cylindrical wall portions extending between the annular ends, and the distance R may be the radius of the tubular structure.

With continued reference to FIGS. 7 and 8, the input device 10 may further include at least one pin 18 and at least one aperture 20. The pin 18 may be fixed to one of the static portion 12 and the movable portion 14, and the aperture 20 may be formed in the other of the static portion 12 and the movable portion 14. For simplicity of explanation, the movable portion 14 includes the pin 18 and the static portion 12 includes the aperture 20 in the illustrated embodiments.

The pin 18 and the aperture 20 are arranged such that a portion of the pin 18 is located within the aperture 20. The pin 18 and the aperture 20 are respectively sized, shaped, and otherwise configured such that the pin 18 is movable within the aperture 20 as the movable portion 14 is displaced relative to the static portion 12.

In operation, contact of the pin 18 with a surface defining the aperture 20 prohibits further displacement of the movable portion 14 in a direction beyond this point of contact as explained in further detail herein. The size and shape of the pin 18 and the aperture 20 may be selected to provide a predetermined range of motion of the movable portion 14 relative to the static portion 12. Accordingly, the provision and arrangement of the pin 18 and aperture 20 provides a means for providing a predetermined range of motion of the handle 140.

The aperture 20 is defined by curved and/or planar surfaces of the static portion 12 or movable portion 14 in which the aperture 20 is formed. The aperture 20 may have a regular or irregular shape.

The size and/or shape of the aperture 20 may be selected such that the movable portion 14 is equally displaceable along each translational axis A₁, A₂. For instance, a width or diameter of the aperture 20 is the same when measured along a first axis parallel to the first translational axis A₁ and along a second axis parallel to the second translational axis A₂. In such an embodiment, the aperture 20 may have a circular shape or have a shape of a regular polygon such as, but not limited to, a square.

Alternatively, the size and/or shape of the aperture 20 may be selected such that the movable portion 14 is displaceable to a greater extent along one translational axis relative to another. For instance, a width or diameter of the aperture 20 is the different when measured along a first axis parallel to the first translational axis A₁ relative to a width or diameter of the aperture 20 measured along a second axis parallel to the second translational axis A₂. In such embodiments, the aperture 20 may have an oval shape or have a shape of an irregular polygon such as, but not limited to, a rectangle or a rhombus.

One of the pin 18 and the aperture 20 is provided with a damping element 22. The damping element 22 is located to prevent direct contact between the pin 18 and the aperture 20.

Two possible arrangements of the damping element 22 are illustrated in FIGS. 7 and 8. In one embodiment, the damping element 22 may be disposed on a portion of the pin 18 located within the aperture 20. In another embodiment, the damping element 22 may be disposed on a surface of the movable portion 14 or the static portion 12 defining the aperture 20. The damping element 22 may have a shape conforming to the shape of the surface of the pin 18 or aperture 20 on which the damping element 22 is disposed.

The damping element 22 is made of a compressible material. The damping element 22 may be compressed in multiple directions such as along an axis parallel to each of the translational axes A₁, A₂ and along an axis parallel to the rotational axis A₃. The damping element 22 may be made of an elastomeric material.

In some embodiments, the aperture 20 is a cavity extending partially into the one of the static portion 12 or movable portion 14. In other embodiments, the aperture 20 is an opening extending fully through a portion of the static portion 12 or movable portion 14. For instance, the aperture 20 has a shape of a counterbore hole that includes a surface on which the damping element 22 is disposed.

A fastening element 24 may be located within the aperture 20 in an adjustable manner. For example, the fastening element 24 may have a threaded element engaged with a threaded surface of the aperture 20. The fastening element 24 may be rotated about the z-axis such that the fastening element 24 is translated along an axis parallel to the rotational axis A₃. Translation of the fastening element 24 may compress the damping element 22. Varying a degree of compression of the damping element 22 may modify the range of the pin 18 within the aperture 20 and may modify the resistance force experienced by the user upon reaching the limit of the range of motion.

In operation, when a user grips the handle 140 and displaces the handle 140 by applying a force thereto, the pin 18 may be directed toward and approach a surface defining the aperture 20. As the movable portion 14 approaches the limit of its range of motion within the aperture 20, the damping element 22 is compressed between the aperture 20 and the pin 18. Compression of the damping element 22 provides a resistance force in a direction substantially opposite to the direction in which the force is applied by the user to the handle 140. Accordingly, this resistance force, which continues to increase as the damping element 22 continues to be compressed, haptically communicates to a user that motion in the direction of the applied force is reaching the end of its possible range.

The input device 10 further comprises sensors, such as accelerometers, gyroscope, or inertial sensors, used to compensate for the effect of gravity.

The input device 10 further comprises a system 30 for tracking displacement of the movable portion 14 relative to the static portion 12. The system for tracking may be a magnetic tracker or an optical tracker.

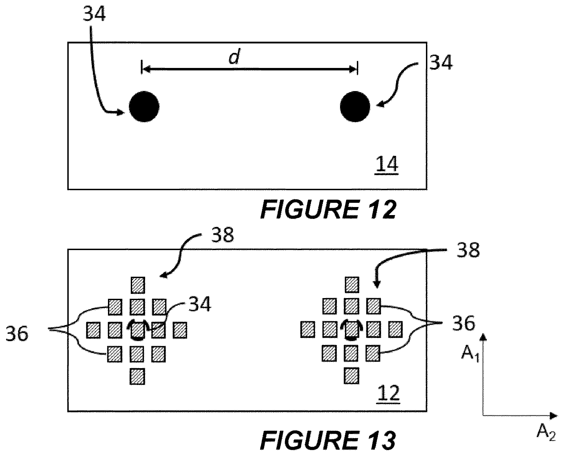

As illustrated in FIGS. 12 and 13, the system 30 is a magnetic tracker that includes a magnetic source 34 configured to generate a magnetic field and an array 38 of sensors 36 configured to measure the generated magnetic field of the magnetic source 34.

The system 30 may include two magnetic sources 34. Each magnetic source 34 is associated with a respective array 38 of sensors 36, which are illustrated in FIG. 12. When the system 110 is assembled, the magnetic source 34 is disposed over the array 38 of the sensors 36 as indicated by dashed lines in FIG. 13.

The magnetic source 34 may be a permanent dipole magnet. The magnetic source may have a cylindrical shape, which is circular in cross-sectional shape as illustrated in FIG. 12, though the person skilled in the art would understand magnets of other shapes could be used.

The magnetic source 34 may be coupled to one of the movable portion 14 and the static part 12, and the sensors 36 are coupled to the other of the movable portion 14 and the static portion 12. For simplicity of description, the system 30 will only be described with respect to an embodiment in which the magnetic source 34 is coupled to the movable portion 14 and the sensors 36 are coupled to the static portion 12.

The magnetic source 34 is coupled to the movable portion 14 such that a direction of magnetization extends substantially perpendicular to the translational axes A₁, A₂.

The magnetic sources 36 are fixed to the movable portion 14 such that the magnetic sources 34 are separated by a fixed distance *d.* The fixed distance *d* may be selected such that the magnetic fields of the respective magnetic sources 34 do not substantially interfere with each other. In some embodiments, the magnetic sources 34 may be coupled to a common substrate or may be coupled to separate substrates otherwise coupled together.

The sensors 36 are arranged in two dimensions. The sensors 36 are separated by a known distance, or pitch, and arranged at known locations (i.e., x and y coordinates). The pitch and locations of the sensors 36 are fixed such that the sensors 36 of a given array 38 are not movable relative to each other.

The pitch of the sensors 36 may be variable. For example, the distance between adjacent sensors 36 may increase as the distance from the geometric center of the array 38 increases. Alternatively, the pitch of the sensors 36 may be constant. In such an embodiment, the distance between adjacent sensors 36 does not change as the distance from the geometric center of the array 38 increases.

The array 38 includes at least three sensors 36. The number of sensors 36 in the input device 10 is selected based on the pre-determined measurement precision needed for the particular application of the input device 10. In addition, increasing the number of sensors enables redundancy.

The sensors 36 may be Hall effect sensors. Hall effect sensors measure the intensity of the magnetic field of the magnetic source 34 and output a voltage that is proportional to that magnetic field measurement.

The sensors 36 may be unidirectional sensors. Unidirectional sensors are configured to measure the magnetic field emitted by the magnetic source 34 in a single direction. In particular, the sensors 36 are configured to measure an intensity of the normal component of the magnetic field, which is the portion of the magnetic field parallel to the direction of magnetization and perpendicular to the x-axis and y-axis of the coordinate system 122 of the of the sensor array 38. Each sensor 36 detects and measures a magnetic field intensity at the respective positions.

In operation, the movable portion 14 of the input device 10 is displaced relative to the static portion 12 when the user applies a force the movable part 141 of the handle 140. As the movable portion 14 is displaced, the magnetic source 34 is displaced. The intensity of the local magnetic field measured by the sensors 36 is used to determine displacement of the magnetic source and, thus, displacement of the movable parts 14, 141 which is communicated to the control unit 300.

While the present disclosure has been described herein with respect to certain illustrated embodiments, those of ordinary skill in the art will recognize and appreciate that it is not so limited. Rather, many additions, deletions, and modifications to the illustrated embodiments may be made without departing from the scope of the disclosure, including legal equivalents thereof. In addition, features from one embodiment may be combined with features of another embodiment while still being encompassed within the scope of the invention as contemplated by the inventors.

## Claims

1. An input device (10) comprising:
a static portion (12);
a movable portion (14), the movable portion (14) being displaceable relative to the static portion (12) along at least two degrees of freedom and up to three degrees of freedom; and
at least three posts (16) extending between the static portion (12) and the movable portion (14), each post (16) being fixed at a first end (17a) to the static portion (12) and being fixed at a second end (17b), opposite the first end, to the movable portion (14), wherein each of the posts (16) extends substantially parallel to each other and extends parallel to an axis (A₃) of rotational freedom when the movable portion (14) and the static portion (12) are in a neutral position,
wherein the movable portion (14) is displaceable along a first axis of translational freedom (A₁), a second axis of translational freedom (A₂), and the axis (A₃) of rotational freedom, and wherein the movable portion (14) and static portion (12) are configured such that, when the movable portion (14) is displaced relative to the static portion (12), any plane (P₁₄) of the movable portion (14) that is perpendicular to the posts (16) in the neutral position and in which at least one of the second ends (17b) of the posts (16) lies remains parallel to any plane (P₁₂) of the static portion (12) that is perpendicular to the posts (16) and in which at least one of the first ends (17a) of the posts (16) lies.

2. The input device (10) of claim 1, wherein each post (16) is elastically deformable.

3. The input device (10) of claim 1 or 2, further comprising a stiffener (26) provided about the first end of each post (16), the stiffener (26) having an abutment surface (28) contactable by the posts (16) when the movable portion (14) is displaced from the neutral position.

4. The input device (10) of claim 3, wherein the stiffener (26) is adjustably coupled to one of the static portion (12) and the movable portion (14).

5. The input device (10) of claim 3 or 4, wherein the abutment surface (28) comprises an arcuate surface and/or a stepped surface.

6. The input device (10) of any of claims 1-5, further comprising:
a pin (18) coupled at a first end to one of the static portion (12) and the movable portion (14); and
an aperture (20) formed in the other of the static portion (12) and the movable portion (14), a second end of the pin (18) received in the aperture (20).

7. The input device (10) of claim 6, further comprising a damping element (22) coupled to one of the pin (18) proximate the second end or on a surface of the static portion (12) or the movable portion (14) defining the opening (20).

8. The input device (10) of claim 6 or 7, wherein, when viewed in a plane perpendicular to posts (16) in the neutral position, the opening (20) has a regular or irregular shape defined by curved surfaces of the static portion (12).

9. The input device (10) of any of claims 1-8, wherein the plurality of posts (16) comprises four posts (16).

10. The input device (10) of any of claims 1-9, wherein a width or diameter (W₁) of each post (16) measured along a first axis parallel to the first translational axis (A₁) is different from a width or diameter (W₂) measured along a second axis parallel to the second translational axis (A₂).

11. The input device (10) of any of claims 1-9, wherein a width or diameter (W₁) of each post (16) measured along a first axis parallel to the first translational axis (A₁) is equal to a width or diameter (W₂) measured along a second axis parallel to the second translational axis (A₂).

12. The input device (10) of any of claims 1-11, wherein each of the plurality of posts (16) is equal in length.

13. The input device (10) of any of claims 1-12, further comprising a system (30) for tracking displacement of the movable portion (14) relative to the static portion (12), the system (30) comprising:
a plurality of sensors (36) coupled to one of the static portion (12) and the movable portion (14); and
at least one magnetic source (34) coupled to the other of the static portion (12) and the movable portion (14), wherein the plurality of sensors (36) are configured to measure an intensity of a magnetic field emitted by the magnetic source (34).

14. The input device (10) of claim 13, wherein the plurality of sensors (36) are unidirectional sensors configured to measure an intensity of the magnetic field of the at least one magnetic source (34) in a direction parallel to the rotational axis (A₃).

15. The input device (10) of any of claims 1-14, wherein the movable portion (14) is displaceable relative to the static portion (12) along two degrees of translational freedom and one degree of rotational freedom.

16. A computer-assisted surgical system (100), comprising:
a robotic arm (110) with a plurality of motorized joints (113); and
a handle (140) for controlling the robotic arm (110), the handle (140) comprising:
a static part (144) coupled to the robotic arm (110);
a movable part (141) coupled to the static part (144) and configured to be displaced relative to the static part (144); and
the input device (10) of any of claims 1-15, wherein the static portion (12) of the input device (10) is disposed within the static part (144) of the handle (140) and the movable portion (14) is disposed within the movable part (141) of the handle (140).
